# EUROPEAN PATENT SPECIFICATION

(11) **EP 2 114 154 B1**
(45) Date of publication and mention of the grant of the patent: **28.08.2013**
(21) Application number: 08725172.4
(22) Date of filing: 05.02.2008
(51) Int. Cl.: A61P 3/06

(54) **METHOD OF TREATING ATHEROSCLEROSIS, DYSLIPIDEMIAS AND RELATED CONDITIONS**
VERFAHREN ZUR BEHANDLUNG VON ATHEROSKLEROSE, HYPERLIPIDÄMIEN UND VERWANDTEN ERKRANKUNGEN
PROCÉDÉ DE TRAITEMENT DE L'ATHÉROSCLÉROSE, DES DYSLIPIDÉMIES ET D'ÉTATS PATHOLOGIQUES APPARENTÉS

(30) Priority: 08.02.2007 US 900254 P
(43) Date of publication of application: 11.11.2009
(73) Proprietor: Merck Sharp & Dohme Corp., Rahway, NJ 07065-0907 (US)
(72) Inventor: PAOLINI, John, F., Rahway, New Jersey 07065-0907 (US); LAI, Eseng, Rahway, New Jersey 07065-0907 (US); MITCHEL, Yale, B., Rahway, New Jersey 07065-0907 (US)
(74) Representative: Hussain, Deeba
(86) International application number: PCT/US2008/001499
(87) International publication number: WO 2008/097535

(56) References cited:
- WO-A1-96/32942
- WO-A1-2004/103370
- WO-A2-2006/089309
- WO-A2-2007/120385
- US-A1- 2004 229 844
- US-A1- 2005 255 158
- MACCUBBIN DARBIE ET AL: "Flushing profile of ER niacin/laropiprant in patients with primary Hypercholesterolemia or mixed dyslipidemia" CIRCULATION, vol. 116, no. 16, Suppl. S, October 2007 (2007-10), page 16, XP009135219 & 80TH ANNUAL SCIENTIFIC SESSION OF THE AMERICAN-HEART-ASSOCIATION; ORLANDO, FL, USA; NOVEMBER 04 -07, 2007 ISSN: 0009-7322
- GLEIM GILBERT ET AL: "Lipid-altering efficacy and safety profile of co-administered extended release niacin/laropiprant and simvastatin in patients with dyslipidemia" CIRCULATION, vol. 116, no. 16, Suppl. S, October 2007 (2007-10), page 127, XP009135220 & 80TH ANNUAL SCIENTIFIC SESSION OF THE AMERICAN-HEART-ASSOCIATION; ORLANDO, FL, USA; NOVEMBER 04 -07, 2007 ISSN: 0009-7322
- DATABASE MEDLINE [Online] US NATIONAL LIBRARY OF MEDICINE (NLM), BETHESDA, MD, US 01 July 2009 MACCUBBIN DARBIE ET AL: 'Flushing profile of extended-release niacin/laropiprant versus gradually titrated niacin extended-release in patients with dyslipidemia with and without ischemic cardiovascular disease.' Database accession no. NLM19576324

## Description

### BACKGROUND OF THE INVENTION

Niacin or nicotinic acid (pyridine-3-carboxylic acid) is a drug commonly known for its effect in elevating serum levels of high density lipoproteins (HDL). However, nicotinic acid is frequently associated with cutaneous vasodilation, sometimes called flushing. This side effect is caused by the nicotinic acid-induced release of prostaglandin D2 in the skin and is so severe that many patients discontinue nicotinic acid treatment.

The present invention relates to the treatment of atherosclerosis, dyslipidemias, and to raising serum HDL levels by administering nicotinic acid in a regimen that achieves therapeutic levels of nicotinic acid rapidly, while reducing or eliminating the cutaneous vasodilation that might otherwise occur, such that treatment can progress to the target dose without substantial flushing. This is achieved in humans by administering nicotinic acid with a compound that antagonizes the DP receptor, namely compound E-Dosing is typically initiated with a one gram (starting) dose of nicotinic acid once daily, in a form that minimizes the flushing effect, followed by a two gram (maintenance) dose, once daily, for the duration of therapy.

One prostaglandin D2 receptor is referred to as "DP" and another prostaglandin D2 receptor is known as "CRTH2". The present invention includes or utilizes selective antagonism of the DP receptor by compound E to prevent, minimize or reduce the flushing that may otherwise occur.

Past dosing titrations have typically begun at sub-therapeutic dosing levels, generally from about 500mg, and escalated over three to four week intervals to a dose that ranges from about 1 gram to as high as 3-4 grams per day. Reaching therapeutic doses in this manner can take several months, and many patients discontinue treatment during the titration phase due to the flushing effect that is experienced. Also, historically patients and physicians are not rigorous in maintaining dose titration schedules, and patients often do not reach target doses because of this failure to titrate WO 2006/089309 discloses a method for treating atherosclerosis wherein nicotinic acid is administered with a DP receptor antagonist with no reference to a specific dosage regiment. The present invention reduces the need to gradually titrate dosage strength over an extended time frame.. The present invention may begin with about an 800mg to 1 gram starting dose of nicotinic acid, once daily, and then advancing to about a 1.6 to about a 2 gram maintenance dose, administered once daily.

Consequently one object of the present invention is to eliminate or reduce substantial flushing (frequency and/or severity) as a side effect during the treatment of humans for atherosclerosis dyslipidemia and in raising serum HDL levels using nicotinic acid while escalating from a therapeutic dose of about 1 gram to about a 2 gram dose more rapidly than has been possible in the past.

Another object of the present invention is to provide combination therapy for atherosclerosis, dyslipidemia and for raising serum HDL levels that minimizes side effects generally.

Yet another object is to provide a fixed combination pharmaceutical composition for oral use that can be used as described herein.

### SUMMARY OF THE INVENTION

The present invention provides a combination of nicotinic acid and Compound E of the following formula: 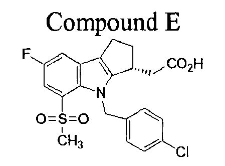 or a pharmaceutically acceptable salt or solvate thereof, for use in treating atherosclerosis, raising serum HDL levels or treating dyslipidemia, in the absence of substantial flushing, wherein about 800mg to about 1 gram of nicotinic acid is administered to a patient once daily for a period ranging from 3-7 days to about 2 months, and thereafter, about 1.6 grams to about 2 grams of nicotinic acid is administered to the patient once daily for the duration of therapy.

Further, the treatment described herein incorporates the administration of simvastatin in an amount that is effective for treating atherosclerosis or dyslipidemias or for lowering LDL.

### DETAILED DESCRIPTION OF THE INVENTION

Niacin or nicotinic acid (pyridine-3-carboxylic acid) is a drug commonly known for its effect in the elevation of high density lipoproteins (HDL) levels, as well as other beneficial alterations of the lipid profile (lowering very low density lipoprotein (VLDL), low density lipoprotein (LDL), triglycerides, free fatty acids (FFA) and lipoprotein(a) [Lp(a)]). Nicotinic acid raises HDL levels when administered to humans in therapeutically effective doses, such as about 500 mg to as high as about 8 grams per day. However, nicotinic acid is frequently associated with cutaneous vasodilation, also called flushing. Flushing typically entails a reddening of the skin, accompanied by warmth, itchiness or irritation. It can be extremely unpleasant, and can be so severe that many patients discontinue nicotinic acid treatment. The present invention relates to the treatment of dyslipidemias and the prevention or reversal of atherosclerosis and to raising serum HDL levels with nicotinic acid with significant attenuation of flushing. This is achieved in humans by administering about an 800 mg to about 1 gram dose of nicotinic acid as a starting dose in the absence of substantial flushing, and thereafter escalating to a single daily dose of about 1.6 grams to about 2 grams of nicotinic acid, in combination with compound E with significant attenuation of flushing, for the duration of therapy. The combination of extended release nicotinic acid with compound E at selectively antagonizes the DP receptor, into a single dosage unit prevents, minimizes or reduces the flushing effect in it frequency and/or severity.

There are at least two receptors that interact with prostaglandin D2, referred to as "DP" and "CRTH2". The DP receptor antagonists that are described herein are selective for the DP receptor over the CRTH2 receptor.

Thus the present invention relates to claim 1

Another aspect of the invention relates to a combination of nicotinic acid and compound E for use in treating atherosclerosis in a human patient in need of such treatment that is comprised of administering to the patient about 800mg to about 1 gram of nicotinic acid and the DP selective receptor antagonist in an amount that is effective to minimize or eliminate substantial flushing, once daily, for a period ranging from a few days to about 2 months, and thereafter, administering to the patient about 2 g of nicotinic acid and the DP selective receptor antagonist, once daily, for the duration of therapy.

Yet another aspect of the invention relates to a combination of nicotinic acid and compound E for use in treating atherosclerosis in a human patient in need of such treatment that is comprised of administering to the patient about 800mg to about I gram of nicotinic acid, the coumpound E in an amount that is effective to minimize or eliminate substantial flushing, and an anti-atherosclerotic effective amount of simvastatin once daily, for a period ranging from a few days to about 2 months, and thereafter, administering to the patient about 2 g of nicotinic acid and the DP selective receptor antagonist, once daily, for the duration of therapy.

Another aspect of the invention that is of interest relates to a combination of nicotinic acid and compound E for use in raising serum HDL levels in a human patient in need of such treatment, comprising administering to the patient a 1 gram dose of nicotinic acid once daily for a few days to a few weeks, and then administering to the patient a 2 gram dose of nicotinic acid once daily for the duration of therapy to raise serum HDL levels in the absence of substantial flushing. To reduce or eliminate such flushing the selective DP receptor antagonist compound E is used. The combination of nicotinic acid and the selective DP receptor antagonist is one means for raising serum HDL levels in the patient in the absence of substantial flushing.

Another aspect of the invention that is of interest relates to a combination of nicotinic acid and compound E for use in treating or preventing dyslipidemia in a human patient in need of such treatment, comprising administering to the patient about 1 gram of nicotinic acid once daily for a few days to a few weeks, and then administering to the patient about 2 grams of nicotinic acid once daily for the duration of therapy to treat dyslipidemia in the absence of substantial flushing. To reduce or eliminate such flushing the selective DP receptor antagonist, compound E is used.

Examples of compounds that selectively antagonize DP receptors are:

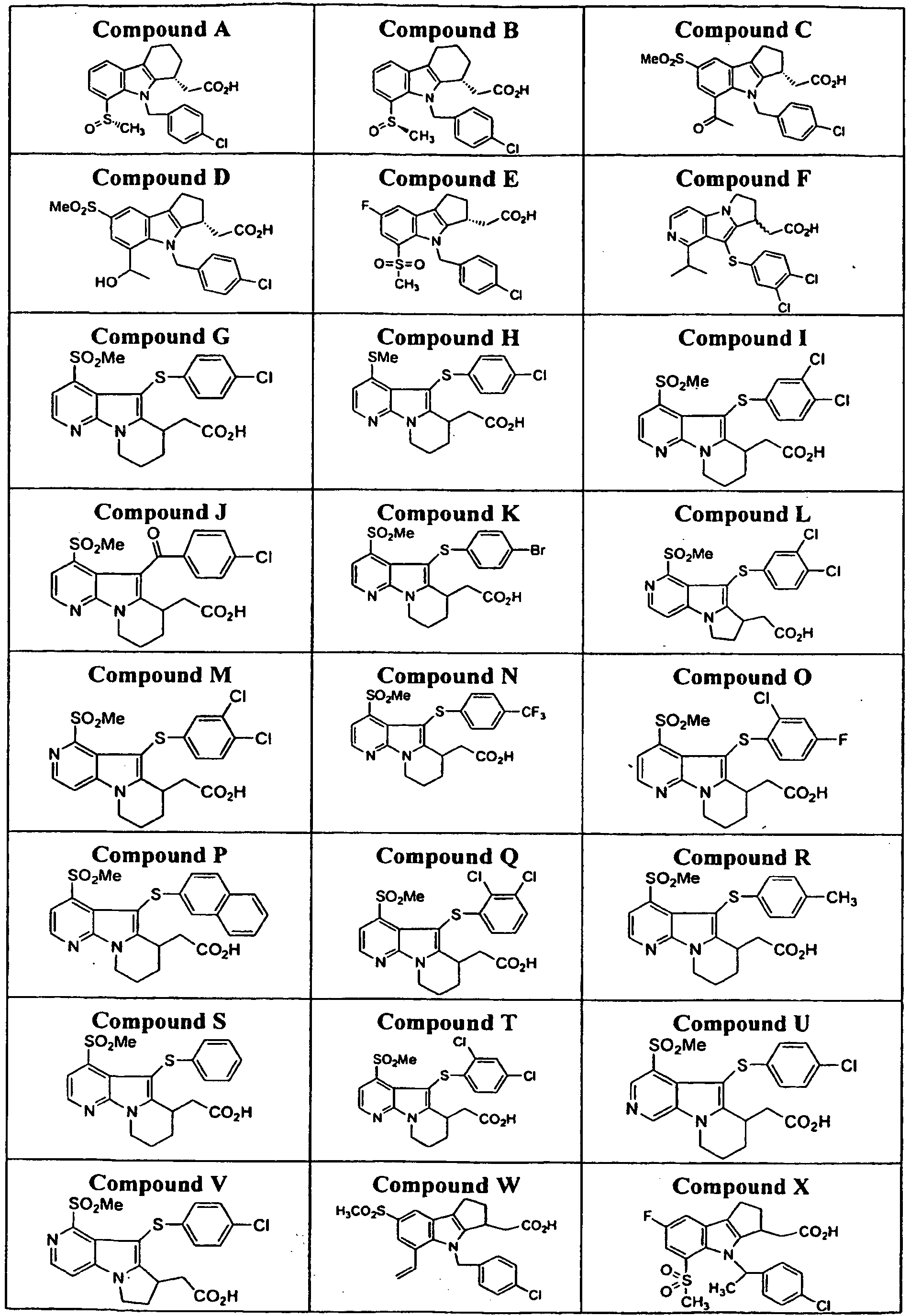

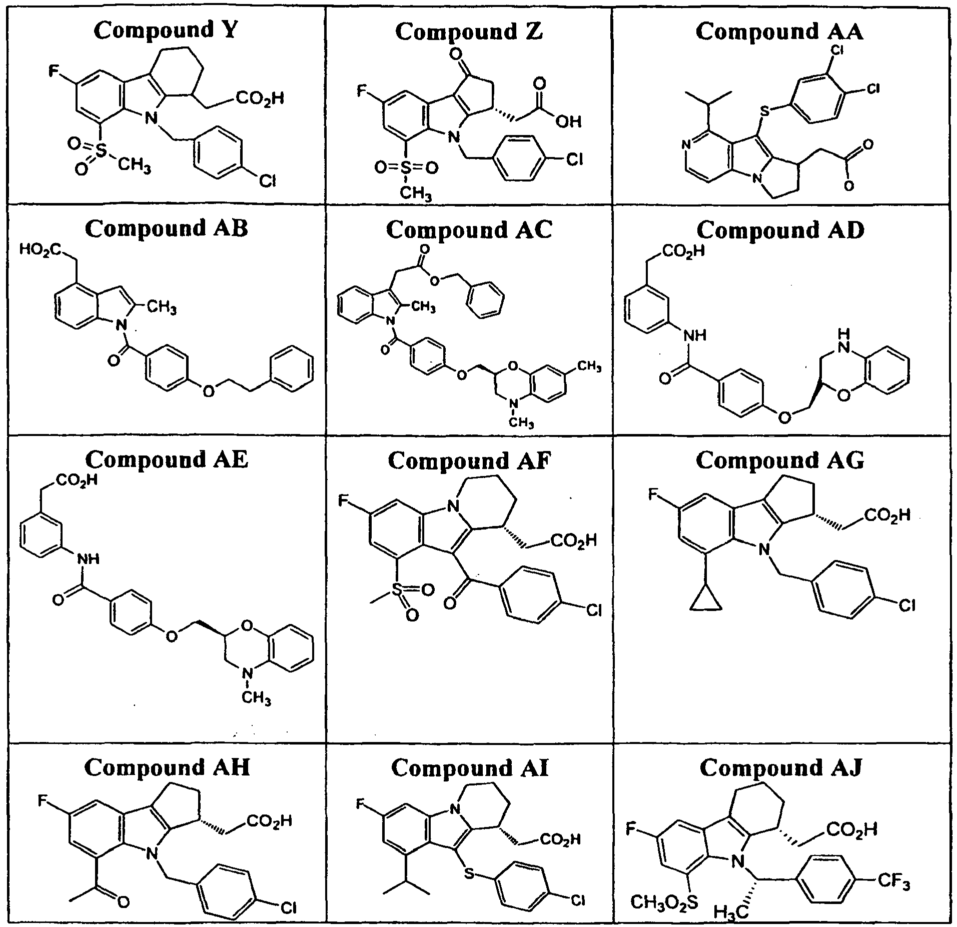

as well as the pharmaceutically acceptable salts and solvates thereof.

Atherosclerosis as used herein refers to a form of vascular disease characterized by the deposition of atheromatous plaques containing cholesterol and lipids on the innermost layer of the walls of large and medium-sized arteries. Atherosclerosis encompasses vascular diseases and conditions that are recognized and understood by physicians practicing in the relevant fields of medicine. Atherosclerotic cardiovascular disease, including restenosis following revascularization procedures, coronary heart disease (also known as coronary artery disease or ischemic heart disease), cerebrovascular disease including multi-infarct dementia, and peripheral vessel disease including erectile dysfunction, are all clinical manifestations of atherosclerosis and are therefore encompassed by the terms "atherosclerosis" and "atherosclerotic disease."

"Dyslipidemia" is used in the conventional sense to refer to abnormal levels of plasma lipids, such as HDL (low), LDL (high), VLDL (high), triglycerides (high), lipoprotein (a) (high), FFA (high) and other serum lipids, or combinations thereof. It may be an uncomplicated condition or part of a particular related disease or condition such as diabetes (diabetic dyslipidemia), metabolic syndrome and the like. Thus, uncomplicated dyslipidemias as well as those that are associated with underlying conditions are included in the present invention.

The term "patient" includes mammals, especially humans, who use the instant active agents for the prevention or treatment of a medical condition. Administering the drugs to the patient includes both self-administration and administration to the patient by another person. The patient may be in need of treatment for an existing disease or medical condition, or may desire prophylactic treatment to prevent or reduce the risk of onset of the disease or condition.

The term "starting dose" refers to an initial dose of nicotinic acid that is considered to be therapeutic for the diseases and conditions described herein, generally about 800mg to about 1 gram. This dose may be administered once daily for a few days, up to a few weeks. As used herein in reference to the duration of usage for the starting dose, "a few days" refers to about 3-7 days.

The term "maintenance dose" refers to the amount of nicotinic acid that is administered to the patient daily after the patient has acclimated to nicotinic acid, generally about 1.6 grams to about 2 grams. Generally the maintenance dose is administered a few days to a few weeks after the initiation of therapy, and is repeated once daily for the duration of therapy.

The term "therapeutically effective amount" is intended to mean that amount of drug that will elicit the desired biological or medical response. As an example, nicotinic acid is often administered at doses from about 1 gram to as high as about 8 grams each day.

The terms "prophylactically effective amount" and "amount that is effective to prevent" refer to that amount of drug that will prevent or reduce the risk of occurrence of the biological or medical event that is sought to be prevented. In many instances, the prophylactically effective amount is the same as the therapeutically effective amount.

The invention described herein includes the administration of the compounds and compositions described herein to prevent or reduce the risk of occurrence, or recurrence where the potential exists, of a coronary heart disease event, a cerebrovascular event, and/or intermittent claudication. Coronary heart disease events are intended to include CHD death, myocardial infarction (i.e., a heart attack), and coronary revascularization procedures. Cerebrovascular events are intended to include ischemic or hemorrhagic stroke (also known as cerebrovascular accidents) and transient ischemic attacks. Intermittent claudication is a clinical manifestation of peripheral vessel disease. The term "atherosclerotic disease event" as used herein is intended to encompass coronary heart disease events, cerebrovascular events, and intermittent claudication experienced one or more non-fatal atherosclerotic disease events are those for whom the potential for recurrence of such an event exists.

Accordingly, the instant invention also provides the combination for use in preventing or reducing the risk of a first or subsequent occurrence of an atherosclerotic disease event comprising the administration of a prophylactically effective amount of the compounds described herein to a patient at risk for such an event while preventing or minimizing substantial flushing. The patient may already have atherosclerotic disease at the time of administration, or may be at risk for developing it.

The use further relates to preventing or slowing new atherosclerotic lesion or plaque formation, and preventing or slowing the progression of existing lesions or plaques, as well as to causing the regression of existing lesions or plaques, while preventing or minimizing substantial flushing.

Accordingly, one aspect of this invention involves the combination for use in halting or slowing the progression of atherosclerosis, including halting or slowing atherosclerotic plaque progression, comprising administering a therapeutically effective amount of compound E in combination with nicotinic acid to a patient in need of such treatment. This use also includes halting or slowing progression of atherosclerotic plaques existing at the time the instant treatment is begun (i.e., "existing atherosclerotic plaques"), as well as halting or slowing formation of new atherosclerotic plaques in patients with atherosclerosis.

Another aspect of this invention involves the combination for use in preventing or reducing the risk of atherosclerotic plaque rupture comprising administering a prophylactically effective amount of compound E along with nicotinic acid to a patient in need of such treatment. Rupture as used herein refers to the breaking loose of plaque, which can become lodged in blood vessels. A further aspect of this invention involves a use for preventing or reducing the risk of developing atherosclerosis, comprising administering a prophylactically effective amount of the compounds described herein to a patient in need of such treatment.

Another aspect of the invention relates to the combination for use in treating or preventing atherosclerosis, dyslipidemias raising serum HDL levels comprising pretreating a human patient in need of such therapy with a flush-inhibiting or reducing effective amount of compound E , thereafter treating said patient with nicotinic acid, a salt or solvate thereof, in an amount that is effective to treat or prevent said atherosclerosis, dyslipidemia or raising serum HDL levels in the absence of substantial flushing.

Yet another aspect of the invention relates to the use described above, further comprising pre-treating or treating the patient with an HMG Co-A reductase inhibitor.

Another aspect of the invention relates to a use of treating or preventing the conditions noted above wherein the HMG Co-A reductase inhibitor is simvastatin.

One aspect of the methods described herein relates to the use of nicotinic acid in an amount that is effective for achieving the results described herein, and compound E. Thus, the DP receptor antagonist has an affinity at the DP receptor (i.e., Kᵢ) that is at least about 10 times higher (a numerically lower Kᵢ value) than the affinity at the CRTH2 receptor. Any compound that selectively interacts with the DP receptor according to these guidelines is deemed "DP selective".

The term "flushing" is defined as reddening, tingling and/or itchiness that are experienced upon the administration of nicotinic acid to a naive patient, i.e., one which has not previously been treated with a therapeutic dose of niacin. Additionally, it includes cutaneous reactions after a patient has been treated in this manner, such as with "breakthrough flushing". "Substantial flushing" is an extent of flushing that is judged by the patient to be uncomfortable or bothersome. It is typically characterized as "severe" or "extreme" flushing according to a global flushing severity score (GFSS) questionnaire.

The GFSS questionnaire seeks to confirm that the patient has taken the medication, then focuses on the flushing components, identifying the number of flushing events that been experienced in the preceding 24 hours (1, 2, 3 or more), overall flushing symptoms reported (0 for none, up to 10 for extremely high). It then queries the patient on duration of the longest flushing event experienced (less than 5 minutes, 5 minutes to 2 hours or greater than 2 hours), the bothersomeness perceived by the patient (0 for not bothersome at all, up to 10 for extremely bothersome), redness (0 for no redness up to 10 for extreme redness), warmth (0 for no warmth experienced, up to 10 for extreme warmth), tingling (0 for no tingling experienced up to 10 for extreme tingling), itching (0 for no itching experienced up to 10 for extreme itching), difficulty sleeping (yes or no) and bothersomeness of the effect as it relates to difficulty sleeping (0 for none up to 10 for extremely bothersome). Slight or mild experiences are calibrated as numbers 1, 2 or 3; moderate reactions are calibrated at 4, 5 or 6; severe experiences are calibrated at 7, 8 or 9, and extreme is number 10.

Hence, for patients that do not experience substantial flushing, such patients have overall GFSS flushing scores from 0 to 6, the upper limit of moderate flushing.

The phrase "in the absence of substantial flushing" refers to patients who are in this 0-6 GFSS range when administered nicotinic acid in therapeutic amounts. The flushing effect of nicotinic acid usually becomes less frequent and less severe as the patient develops tolerance to the drug at therapeutic doses, but the flushing effect still occurs to some extent. Thus, "in the absence of substantial flushing" refers to the reduced severity of flushing when it occurs, or fewer flushing events than would otherwise occur as measured by the GFSS. Preferably, the incidence of flushing is reduced by at least about a third, more preferably the incidence is reduced by half, and most preferably, the flushing incidence is reduced by about two thirds or more. Likewise, the severity is preferably reduced by at least about a third, more preferably by at least half, and most preferably by at least about two thirds. Clearly a one hundred percent reduction in flushing incidence and severity is most preferable, but is not required.

The specific dosage amounts for any particular patient will depend upon a variety of factors including the age, body weight, general health, sex, diet, time of administration, rate of excretion, drug combination and the severity of the patient's condition. Consideration of these factors is well within the purview of the ordinarily skilled clinician for the purpose of determining the therapeutically effective or prophylactically effective dosage amount needed to prevent, counter, or arrest progress of the condition. It is expected that the compounds described herein will be administered on a daily basis for a length of time appropriate to treat or prevent the medical condition relevant to the patient, including a course of therapy lasting months, years or the life of the patient.

One or more additional active agents may be administered with the compounds described herein. The additional active agent or agents can be lipid modifying compounds or agents having other pharmaceutical activities, or agents that have both lipid-modifying effects and other pharmaceutical activities. Examples of additional active agents which may be employed include to HMG-CoA reductase inhibitors, which include statins in their lactonized or dihydroxy open acid forms and pharmaceutically acceptable salts and esters thereof, including lovastatin (see US Patent No. 4,342,767), simvastatin (see US Patent No. 4,444,784), dihydroxy open-acid simvastatin, particularly the ammonium or calcium salts thereof, pravastatin, particularly the sodium salt thereof (see US Patent No. 4,346,227), fluvastatin particularly the sodium salt thereof (see US Patent No. 5,354,772), atorvastatin, particularly the calcium salt thereof (see US Patent No. 5,273,995), pitavastatin also referred to as NK-104 (see PCT international publication number WO 97/23200) and rosuvastatin, also known as ZD-4522, (CRESTOR^{®}; see US Patent No. 5,260,440); HMG-CoA synthase inhibitors; squalene epoxidase inhibitors; squalene synthetase inhibitors (also known as squalene synthase inhibitors), acyl-coenzyme A: cholesterol acyltransferase (ACAT) inhibitors including selective inhibitors of ACAT-1 or ACAT-2 as well as dual inhibitors of ACAT-1 and - 2; microsomal triglyceride transfer protein (MTP) inhibitors; endothelial lipase inhibitors; bile acid sequestrants; LDL receptor inducers; platelet aggregation inhibitors, for example glycoprotein IIb/IIIa fibrinogen receptor antagonists and aspirin; human peroxisome proliferator activated receptor gamma (PPARγ) agonists including the compounds commonly referred to as glitazones for example pioglitazone and rosiglitazone and, including those compounds included within the structural class known as thiazolidine diones as well as those PPARγ agonists outside the thiazolidine dione structural class; PPARα agonists such as clofibrate, fenofibrate including micronized fenofibrate, and gemfibrozil; PPAR dual α/γ agonists; vitamin B₆ (also known as pyridoxine) and the pharmaceutically acceptable salts thereof such as the HCl salt; vitamin B₁₂ (also known as cyanocobalamin); folic acid or a pharmaceutically acceptable salt or ester thereof such as the sodium salt and the methylglucamine salt; anti-oxidant vitamins such as vitamin C and E and beta carotene; beta-blockers; angiotensin II antagonists such as losartan; angiotensin converting enzyme inhibitors such as enalapril and captopril; renin inhibitors, calcium channel blockers such as nifedipine and diltiazem; endothelin antagonists; agents that enhance ABCA1 gene expression; cholesteryl ester transfer protein (CETP) inhibiting compounds, 5-lipoxygenase activating protein (FLAP) inhibiting compounds, 5-lipoxygenase (5-LO) inhibiting compounds, farnesoid X receptor (FXR) ligands including both antagonists and agonists; Liver X Receptor (LXR)-alpha ligands, LXR-beta ligands, bisphosphonate compounds such as alendronate sodium; cyclooxygenase-2 inhibitors such as rofecoxib and celecoxib; and compounds that attenuate vascular inflammation.

Cholesterol absorption inhibitors can also be used in the present invention. Such compounds block the movement of cholesterol from the intestinal lumen into enterocytes of the small intestinal wall, thus reducing serum cholesterol levels. Examples of cholesterol absorption inhibitors are described in U.S. Patent Nos. 5,846,966, 5,631,365, 5,767,115, 6,133,001, 5,886,171, 5,856,473, 5,756,470, 5,739,321, 5,919,672, and in PCT application Nos. WO 00/63703, WO 00/60107, WO 00/38725, WO 00/34240, WO 00/20623, WO 97/45406, WO 97/16424, WO 97/16455, and WO 95/08532. The most notable cholesterol absorption inhibitor is ezetimibe, also known as 1-(4-fluorophenyl)-3(R)-[3(S)-(4-fluorophenyl)-3-hydroxypropyl)]-4(S)-(4-hydroxyphenyl)-2-azetidinone, described in U.S. Patent Nos. 5,767,115 and 5,846,966.

Therapeutically effective amounts of cholesterol absorption inhibitors include dosages of from about 0.01 mg/kg to about 30 mg/kg of body weight per day, preferably about 0.1 mg/kg to about 15 mg/kg.

For diabetic patients, the compounds used in the present invention can be administered with conventional diabetic medications. For example, a diabetic patient receiving treatment as described herein may also be taking insulin or an oral antidiabetic medication. One example of an oral antidiabetic medication useful herein is metformin.

### Dosage Information

Nicotinic acid as used herein refers to pyridine-3-carboxylic acid. However, salts and solvates of nicotinic acid are also included for use in the present invention, and numerous pharmaceutically acceptable salts and solvates of nicotinic acid are useful in the present invention. Alkali metal salts, in particular, sodium and potassium, form salts that are useful as described herein. Likewise alkaline earth metals, in particular, calcium and magnesium, form salts that are useful as described herein. Various salts of amines, such as ammonium and substituted ammonium compounds also form salts that are useful as described herein. Similarly, solvated forms of nicotinic acid are useful within the present invention. Examples include the hemihydrate, mono-, di-, tri- and sesquihydrate. Of particular interest for use in the present invention is the free acid, pyridine-3-carboxylic acid.

DP antagonists, as described herein, are useful for reducing or preventing the flushing effect in mammalian patients, particularly humans, at dosages ranging from as low as about 0.01 mg/kg/day to as high as about 100 mg/kg/day, administered in single or divided daily doses. Preferably the dosages are from about 0.1 mg/day to as high as about 1.0 g/day, in single or divided daily doses. Examples of doses for selective DP receptor antagonists include: 1 mg, 2mg, 2.5mg, 5mg, 10mg, 15mg, 20mg, 25mg, 30mg, 40mg, 50mg, 60mg, 75mg, 80mg, 90mg, 100mg, 120mg, 125mg, 150mg, 175mg, 200mg, 250mg, 300mg, 400mg, 500mg and the like.

Routine dosing of nicotinic acid ranges from as low as about 50 mg/day to as high as about 8 g/day, in single or divided daily doses. Lower dosages can be used initially, and dosages increased to further minimize the flushing effect. The present invention utilizes a regimen that includes about 800mg to about 1 gram as a starting dose followed by about 1.6 grams to about 2 grams as a maintenance dose for the duration of therapy.

The compounds used in the present invention can be administered via any conventional route of administration. The preferred route of administration is oral.

The nicotinic acid and the DP antagonist can be administered together or sequentially in single or multiple daily doses, e.g., bid, tid or qid, without departing from the invention. If particularly long sustained release is desired, such as a sustained release product showing a release profile that extends beyond 24 hours, dosages may be administered every other day. However, single daily doses are preferred. Likewise, morning or evening dosages can be utilized. Preferably evening or nighttime dosing is utilized.

### Pharmaceutical Compositions

The typical pharmaceutical compositions described herein are generally comprised of nicotinic acid, compound E and a pharmaceutically acceptable carrier.

Examples of suitable oral compositions include tablets, capsules, troches, lozenges, suspensions, dispersible powders or granules, emulsions, syrups and elixirs. Examples of carrier ingredients include diluents, binders, disintegrants, lubricants, sweeteners, flavors, colorants, preservatives, and the like. Examples of diluents include, for example, calcium carbonate, sodium carbonate, lactose, calcium phosphate and sodium phosphate. Examples of granulating and disintegrants include corn starch and alginic acid. Examples of binding agents include starch, gelatin and acacia. Examples of lubricants include magnesium stearate, calcium stearate, stearic acid and talc. The tablets may be uncoated or coated by known techniques. Such coatings may delay disintegration and thus, absorption in the gastrointestinal tract and thereby provide a sustained action over a longer period.

In one embodiment of the invention, about 800mg to about 1 gram of nicotinic acid is combined with compound E and the carrier to form a fixed combination product. This fixed combination product may be a tablet or capsule for oral use.

More particularly, in another embodiment of the invention, about 800 mg to about 1 gram of nicotinic acid and about 1 to 500 mg of the DP antagonist are combined with the pharmaceutically acceptable carrier, providing a tablet or capsule for oral use.

Even more particularly, in another embodiment of the invention, about 800 mg to about 1 gram of nicotinic acid and about 1 to 100 mg of the selective DP receptor antagonist are combined with the pharmaceutically acceptable carrier, providing a tablet or capsule for oral use.

Still more particularly, one embodiment of the invention relates to the once daily administration of a fixed combination that is comprised of about 800 mg to about 1 gram of nicotinic acid, about 20 mg of the selective DP receptor antagonist and further optionally containing about 20mg of simvastatin, for a period ranging from about 3 days to about 2 months, followed by the administration once daily of two fixed combination dosage forms, comprising in total about 1.6 grams to about 2 grams of nicotinic acid, about 40 mg of a selective DP receptor antagonist and further optionally containing about 40 mg of simvastatin for the duration of therapy, in the absence of substantial flushing.

Sustained release over a longer period of time may be particularly important in the formulation of nicotinic acid pharmaceutical compositions. Sustained release tablets are particularly preferred. For example, a time delay material such as glyceryl monostearate or glyceryl distearate may be employed. The dosage form may also be coated by the techniques described in the U.S. Patent Nos. 4,256,108; 4,166,452 and 4,265,874 to form osmotic therapeutic tablets for controlled release.

Other controlled release technologies are also available. Typical ingredients that are useful to slow the release of nicotinic acid in sustained release tablets include various cellulosic compounds, such as methylcellulose, ethylcellulose, propylcellulose, hydroxypropylcellulose, hydroxyethylcellulose, hydroxypropylmethylcellulose, microcrystalline cellulose, starch and the like. Various natural and synthetic materials are also of use in sustained release formulations. Examples include alginic acid and various alginates, polyvinyl pyrrolidone, tragacanth, locust bean gum, guar gum, gelatin, various long chain alcohols, such as cetyl alcohol and beeswax.

A sustained release tablet that is of particular interest utilizes nicotinic acid in combination with one or more of the cellulosic compounds noted above, compressed into a sustained release tablet to form a polymer matrix. The DP antagonist compound can be incorporated into the blend before compression, or can be coated onto the outer surface of the matrix or layered onto an outer surface of the matrix.

In an embodiment that is of particular interest, the nicotinic acid and matrix-forming material are combined and compressed to form a sustained release layer, and the DP antagonist compound is blended with one or more agents and coated onto the outer surface of the core or layered onto an outer surface of the nicotinic acid-containing layer.

Optionally and of even more interest is a tablet as described above, further containing an HMG Co-A reductase inhibitor, for example, simvastatin. This particular embodiment thus contains three active ingredients, the HMG Co-A reductase inhibitor and the selective DP receptor antagonist, which may be releasable from the first layer substantially upon ingestion, and the nicotinic acid which may be releasable over a longer period of time.

Typical release time frames for sustained release tablets in accordance with the present invention range from about 1 to as long as about 48 hours, preferably about 4 to about 24 hours, and more preferably about 8 to about 16 hours.

In another embodiment that is of particular interest, the nicotinic acid and matrix-forming material are combined and compressed to form a sustained release portion, and the selective DP receptor antagonist compound is blended with one or more excipients, and a bilayer tablet is produced. The layers can have different dissolution profiles, such that the nicotinic acid layer dissolves over an extended period of time, such as from about 1-48 hours, while the DP receptor antagonist containing layer dissolves more rapidly, from about 10-15 minutes to as long as about 3-6 hours. Coating agents can be used to modulate the dissolution rates for the layers if desired, and to esthetically mask the bilayer nature of the tablet.

Optionally and of even more interest is a tablet as described above, further incorporating an HMG Co-A reductase inhibitor, for example, simvastatin. This particular embodiment thus contains three active ingredients, the HMG Co-A reductase inhibitor and the DP antagonist, which may be releasable substantially upon ingestion, and the nicotinic acid which may be releasable over a longer period of time as described above.

The nicotinic acid receptor has been identified and characterized in WO02/084298A2 published on October 24, 2002 and in Soga, T. et al., Tunaru, S. et al. and Wise, A. et al. (citations above). through AH is disclosed in WO2004/103370A1 published on 2 December 2004 and the references cited therein. For example, DP receptor antagonists can be obtained in accordance with WO01/79169 published on October 25, 2001, EP 1305286 published on May 2, 2003, WO02/094830 published on November 28, 2002 and WO03/062200 published on July 31, 2003.

Compound AB can be synthesized in accordance with the description set forth in WO01/66520A1 published on September 13, 2001; Compound AC can be synthesized in accordance with the description set forth in WO03/022814A1 published on March 20, 2003, and Compounds AD and AE can be synthesized in accordance with the description set forth in WO03/078449 published on September 25, 2003.

One particularly preferred example for use in the present invention relates to a bilayer tablet, a first layer of which contains about 1 gram of nicotinic acid in a sustained release matrix, and a second layer of which contains about 20 mg of the DP receptor antagonist.

Another particularly preferred example for use in the present invention relates to a bilayer tablet, layer one of which contains about I gram of nicotinic acid in a sustained release matrix, and layer two of which contains about 20 mg of the DP receptor antagonist and about 10mg, 20mg or 40mg of simvastatin.

One aspect of the invention described herein relates to a use described above wherein one tablet as described above, is administered to the patient once daily, for a period of time sufficient to acclimate to any residual flushing effect from the nicotinic acid, and then escalating to two tablets taken once daily for the duration of therapy. This regimen is surprising in that patients can rapidly begin to utilize a two gram maintenance dose of nicotinic acid, while experiencing minimal flushing.

### Biology

The compounds used in the present invention that function as selective DP antagonists typically demonstrate an affinity (Kᵢ) for DP that is at least about 10 times higher (a numerically lower Kᵢ value) than the affinity (Kᵢ) for CRTH2 receptors. Typical DP antagonists are at least about 10-fold selective for the DP receptor over the CRTH2 receptor. More particularly, the selective DP receptor antagonist is at least about 100 fold selective for the DP receptor relative to the CRTH2 receptor. Even more particularly, the DP selective antagonist compound is at least about 800-1000 fold selective for the DP receptor over the CRTH2 receptor, i..e., the affinity (Kᵢ) for the DP receptor is 800-1000 times higher than the affinity (Kᵢ) for the CRTH2 receptor.

As used herein when a compound "selectively modulates the DP receptor", the compound binds to and antagonizes the DP receptor at a concentration that is achievable at therapeutic doses, while not substantially modulating the CRTH2 receptor at such therapeutically achievable concentrations.

Generally the DP antagonists have an affinity (Kᵢ) for the CRTH2 receptor of about 0.5 micromolar or higher. Compounds having a binding affinity for CRTH2 of about 0.5 micromolar or higher, and a selectivity for the DP receptor over CRTH2 of at least about 10 fold, are useful to inhibit the flushing effect seen when nicotinic acid is administered without such selective DP antagonists.

### Determination of the Affinity and Selectivity of Compounds at Recombinant Human DP and CRTH2 Receptors

The receptor affinity and selectivity of compounds at DP and CRTH2 was determined using radioligand binding assays as described in Abramovitz M, et al. Biochem. Biophys. Acta (2000)1483: 285-293, and Sawyer N, et al. Br. J. Pharmacol. (2002); 137: 1163-1172. Briefly, stable cell lines that individually express human DP and CRTH2 receptors were established using human embryonic kidney (HEK) 293EBNA (Epstein Barr virus Nuclear Antigen) cells (designated HEK293E cell lines). Membrane fractions prepared from these recombinant cell lines were employed in equilibrium competition radioligand binding assays to determine the affinity and selectivity of compounds at the DP and CRTH2 receptors.

DP and CRTH2 cDNAs corresponding to full length coding sequences were subcloned into the appropriate sites of the mammalian expression vector pCEP4 (Invitrogen) and expressed in HEK293E cells. Membranes were prepared by differential centrifugation (1000 x g for 10 min, then 160,000 x g for 30 min, all at 4°C) following lysis of the cells by nitrogen cavitation at 800 psi for 30 min on ice in the presence of protease inhibitors (2 mM AEBSF, 10 µM E-64, 100 µM leupeptin and 0.05 mg/mL pepstatin). The 160,000 x g pellets were resuspended in 10 mM HEPES/KOH (pH 7.4) containing 1 mM EDTA at approximately 5 to 10 mg/mL protein by Dounce homogenisation (Dounce A; 10 strokes), frozen in liquid nitrogen and stored at -80°C. Receptor binding assays were performed in a final incubation volume of 0.2 mL in 10 mM HEPES/KOH (pH 7.4), containing 1 mM EDTA, 10 mM MnCl₂ and 0.7 nM [³H]PGD₂ (200 Ci/mmol). The reaction was initiated by addition of membrane protein (approximately 30 µg for DP and 10 µg for CRTH2) from the 160,000 x g fraction. Ligands were added in dimethylsulfoxide (DMSO) which was kept constant at 1 % (v/v) in all incubations. Non-specific binding was determined in the presence of 10 µM of non-radioactive PGD₂. Incubations were conducted on a mini-orbital shaker at room temperature for 60 min. The binding assay was terminated by rapid filtration through a 96-well Unifilter GF/C (Canberra Packard) prewetted in assay incubation buffer without EDTA (at 4°C) using a Tomtec Mach III 96-well semi-automated cell harvester. The filters were washed with 3 to 4 mL of the same buffer, dried for 90 min at 55°C and the residual radioactivity bound to the individual filters determined by scintillation counting with addition of 50 µL of Ultima Gold F (Canberra Packard) using a 1450 MicroBeta (Wallac) counter.

Maximum specific binding was defined as the total binding minus the non-specific binding in the absence of competitor. Specific binding was determined at each concentration of compound and was expressed as a percentage of the maximum specific binding. Sigmoidal equilibrium competition curves were constructed by expressing percentage maximum specific binding as a function of test compound concentration and analyzed by a custom designed software package employing a simplex driven non-linear least-squares curve fitting routine based on a four parameter equation to determine the inflection point (InPt). The binding affinity of the test compound was determined by calculating the equilibrium inhibition constant (Kᵢ) from the equation Kᵢ = InPt/1+([radioligand]/K_{d}), where K_{d} is the equilibrium dissociation constant for the radioligand-receptor interaction. When InPt could not be determined the IC₅₀ was used (i.e. the concentration of test compound required to inhibit 50 % of the maximum specific binding).

Generally the compounds disclosed herein demonstrate a Kᵢ for the DP receptor of from about as low as about 0.4 nM to as high as about 16.3 nM. Likewise, the compound used in the present invention generally demonstrate a Kᵢ for the CRTH2 receptor of as low as about 180 nM to as high as about 22,000 nM or even higher.

### Effect of Compounds on Nicotinic acid-Induced Vasodilation in Mice (Reference example)

The potency of the selective DP antagonists described herein can be demonstrated using a murine model of human nicotinic acid-induced flushing, measuring the flushing inhibitory effect. Blood flow in the mouse ear (a measure of vasodilation, a prominent component of flushing in humans) is measured after administration of nicotinic acid to mice that had been pretreated with vehicle (as a control) or a DP antagonist. Specifically, male C57BL/6 mice (∼25 g) were used in the study. Five mice were evaluated in each test group. Nembutal was diluted with water to a final concentration of 5 mg/ml and injected 0.3 ml/mouse intraperitoneally. DP antagonists were dissolved in 5% hydroxypropyl β-cyclodextrin at a final concentration of 5 mg/ml and the compounds were administered intraperitoneally at a volume of 0.2 ml/mouse (∼40 mpk). Nicotinic acid was dissolved in 5% hydroxypropyl β-cyclodextrin at a final concentration of 12.5 mg/ml. The nicotinic acid stock solution was adjusted to pH 7.4 with 2 N NaOH and injected 0.2 ml/mouse subcutaneously (∼100 mpk).

Perfusion of mouse ear skin was monitored with a laser Doppler perfusion imager (PeriScan PIM II, Primed, Sweden) every 30 seconds for 15 minutes starting 5 minutes prior to nicotinic acid administration. Percent changes in mean perfusion over the 10 minute period after vehicle or nicotinic acid administration were calculated and a graph of percent change in mean perfusion vs. time was generated for each animal. The area under the curve (AUC) of mean perfusion (% Δ x min) was then calculated from each graph and the results are expressed in mean AUC ± SEM for each group.

Compound D suppressed PGD-2 induced vasodilation in the mouse. The DP antagonists tested suppressed nicotinic acid-induced vasodilation in the mouse.

### Clinical Trial of 1 gram Extended Release Niacin With and Without Selective DP Receptor Antagonist (Compound E)

This trial demonstrated the benefit of the selective DP receptor antagonist compound, compound E, on extended-release niacin (ERN) induced flushing.

### Methods:

Patients (N = 412) were randomized to ERN 1 gram (given as Niaspan®, with no prior titration) plus compound E placebo, 18.75, 37.5, 75 or 150mg, or double placebo for 4 weeks (acute phase) with doubling of the respective doses for the remaining 4 weeks (chronic phase). Patients reported flushing intensity using the validated Global Flushing Severity Score (GFSS); none/mild (0-3), moderate (4-6), severe (7-9) and extreme (10) in a daily eDiary. A subset of patients was randomized to compound E 150mg versus placebo in a 4-week crossover safety study.

### Results:

Patients on Compound E plus ERN reported significantly lower acute (dose-response p<0.001) and chronic phase (dose-response p=0.021) flushing intensity compared to ERN alone. At each coadministered dose of Compound E, there was a significantly (p<0.05) lower flushing intensity versus ERN alone. Compound E (150mg) did not significantly impact the lipid altering effects of ERN. There was a low incidence of adverse experiences with Compound E, either alone or coadministered with ERN.

In conclusion, patients receiving Compound E had significantly lower flushing intensity associated with ERN treatment. Compound E significantly improved both tolerability and achievement of optimal therapeutic dosing of niacin.

## Claims

1. A combination of nicotinic acid and Compound E of the following formula: 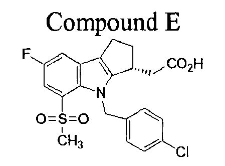 or a pharmaceutically acceptable salt or solvate thereof, for use in treating atherosclerosis, raising serum HDL levels or treating dyslipidemia, in the absence of substantial flushing, wherein about 800mg to about 1 gram of nicotinic acid is administered to a patient once daily for a period ranging from 3-7 days to about 2 months, and thereafter, about 1.6 grams to about 2 grams of nicotinic acid is administered to the patient once daily for the duration of therapy.

2. A combination for use of claim 1 further comprising simvastatin.

3. A combination for use of claim 1 or 2, wherein the nicotinic acid ranging from about 800 mg to about 1 gram is in the form of a sustained release bilayer tablet, comprised of a first layer containing nicotinic acid in a sustained release matrix, and a second layer containing an effective amount of Compound E.

4. A combination for use of claim 1 or 2, wherein the 1.6 grams to about 2 grams of nicotinic acid is in the form of two sustained release bilayer tablets, each comprised of a first layer containing about 800 mg to about 1 gram of nicotinic acid, and a second layer containing an effective amount of Compound E

5. A combination for use of claim 3 or 4, wherein the second layer further contains about 20 mg of simvastatin.

6. A combination for use of claim 3 or 4, wherein the second layer further contains an effective amount of atorvastatin.

7. A combination for use of claim 1 further comprising an HMG-CoA reductase inhibitor.

8. A combination for use of claim 1, wherein the combination is in the form of a bilayer tablet, comprised of a first layer containing about 1g of nicotinic acid in a sustained release matrix, and a second layer containing about 20mg of Compound E.

9. A combination for use of claim 1, wherein the combination is in the form of a bilayer tablet, comprised of a first layer containing about 1g of nicotinic acid in a sustained release matrix, and a second layer containing about 20mg of Compound E and about 10mg, 20mg or 40mg of simvastatin.

10. A combination for use of claim 1, wherein the use comprises once daily administration of a fixed combination that is comprised of about 800mg to about 1 gram of nicotinic acid, about 20mg of compound E and optionally about 20mg of simvastatin, for a period ranging from about 3 days to about 2 months, followed by once daily administration of two fixed combination dosage forms, comprising in total about 1.6 grams to about 2 grams of nicotinic acid, about 40mg of compound E and optionally 40mg of simvastatin, for the duration of therapy.

11. The use of a combination of nicotinic acid and Compound E of the following formula: 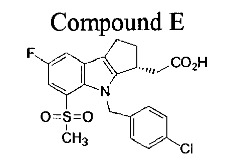 or a pharmaceutically acceptable salt or solvate thereof, for the manufacture of a medicament for treating atherosclerosis, wherein about 800mg to about 1 gram of nicotinic acid and compound E is administered to a patient once daily for a period ranging from 3-7 days to about 2 months, and thereafter, about 2 grams of nicotinic acid and compound E is administered to the patient once daily for the duration of therapy, wherein compound E is administered in an amount that is effective to minimize or eliminate substantial flushing.

12. The use of claim 11 further comprising an HMG-CoA reductase inhibitor.

13. The use of claim 11, wherein the combination is in the form of a bilayer tablet, comprised of a first layer containing about 1g of nicotinic acid in a sustained release matrix, and a second layer containing about 20mg of Compound E.

14. The use of claim 11, wherein the combination is in the form of a bilayer tablet, comprised of a first layer containing about 1g of nicotinic acid in a sustained release matrix, and a second layer containing about 20mg of Compound E and about 10mg, 20mg or 40mg of simvastatin.

15. The use of claim 11, wherein the use comprises once daily administration of a fixed combination that is comprised of about 800mg to about 1 gram of nicotinic acid, about 20mg of compound E and optionally about 20mg of simvastatin, for a period ranging from about 3 days to about 2 months, followed by once daily administration of two fixed combination dosage forms, comprising in total about 2 grams of nicotinic acid, about 40mg of compound E and optionally 40mg of simvastatin, for the duration of therapy.

## Patentansprüche

1. Eine Kombination aus Nikotinsäure und Verbindung E der folgenden Formel: 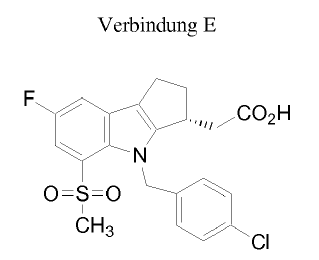 oder einem pharmazeutisch annehmbaren Salz oder Solvat davon zur Verwendung bei der Behandlung von Atherosklerose, zur Erhöhung von Serum-HDL-Spiegeln oder zur Behandlung von Dyslipidämie, ohne dass eine bedeutende Hautgefäßerweiterung auftritt, wobei etwa 800 mg bis etwa 1 Gramm Nikotinsäure einmal täglich für einen Zeitraum im Bereich von 3 - 7 Tagen bis etwa 2 Monaten an einen Patienten verabreicht werden und anschließend etwa 1,6 Gramm bis etwa 2 Gramm Nikotinsäure einmal täglich für die Dauer der Therapie an den Patienten verabreicht werden.

2. Eine Kombination zur Verwendung nach Anspruch 1, die ferner Simvastatin umfasst.

3. Eine Kombination zur Verwendung nach Anspruch 1 oder 2, wobei die Nikotinsäure im Bereich von etwa 800 mg bis etwa 1 Gramm in Form einer 2-Schicht-Tablette zur Freisetzung mit konstanter Geschwindigkeit, umfassend eine erste Schicht, die Nikotinsäure in einer Matrix zur Freisetzung mit konstanter Geschwindigkeit enthält, und eine zweite Schicht, die eine wirksame Menge an Verbindung E enthält, vorliegt.

4. Eine Kombination zur Verwendung nach Anspruch 1 oder 2, wobei die 1,6 Gramm bis etwa 2 Gramm Nikotinsäure in Form von zwei 2-Schicht-Tabletten zur Freisetzung mit konstanter Geschwindigkeit vorliegen, welche jeweils eine erste Schicht, die etwa 800 mg bis etwa 1 Gramm Nikotinsäure enthält, und eine zweite Schicht, die eine wirksame Menge an Verbindung E enthält, umfassen.

5. Eine Kombination zur Verwendung nach Anspruch 3 oder 4, wobei die zweite Schicht ferner etwa 20 mg Simvastatin enthält.

6. Eine Kombination zur Verwendung nach Anspruch 3 oder 4, wobei die zweite Schicht ferner eine wirksame Menge an Atorvastatin enthält.

7. Eine Kombination zur Verwendung nach Anspruch 1, ferner umfassend einen HMG-CoA-Reduktase-Inhibitor.

8. Eine Kombination zur Verwendung nach Anspruch 1, wobei die Kombination in Form einer 2-Schicht-Tablette vorliegt, umfassend eine erste Schicht, die etwa 1 g Nikotinsäure in einer Matrix zur Freisetzung mit konstanter Geschwindigkeit enthält, und eine zweite Schicht, die etwa 20 mg Verbindung E enthält.

9. Eine Kombination zur Verwendung nach Anspruch 1, wobei die Kombination in Form einer 2-Schicht-Tablette vorliegt, umfassend eine erste Schicht, die etwa 1 g Nikotinsäure in einer Matrix zur Freisetzung mit konstanter Geschwindigkeit enthält, und eine zweite Schicht, die etwa 20 mg Verbindung E und etwa 10 mg, 20 mg oder 40 mg Simvastatin enthält.

10. Eine Kombination zur Verwendung nach Anspruch 1, wobei die Verwendung die einmal tägliche Verabreichung einer feststehenden Kombination, die etwa 800 mg bis etwa 1 Gramm Nikotinsäure, etwa 20 mg Verbindung E und gegebenenfalls etwa 20 mg Simvastatin umfasst, über einen Zeitraum, der von etwa 3 Tagen bis etwa 2 Monate reicht, gefolgt von der einmal täglichen Verabreichung von zwei feststehenden Kombinationsdosisformen, die insgesamt etwa 1,6 Gramm bis etwa 2 Gramm Nikotinsäure, etwa 40 mg Verbindung E und gegebenenfalls 40 mg Simvastatin umfassen, für die Dauer der Therapie umfasst.

11. Die Verwendung einer Kombination aus Nikotinsäure und Verbindung E der folgenden Formel: 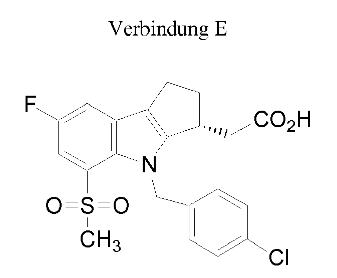 oder einem pharmazeutisch annehmbaren Salz oder Solvat davon zur Herstellung eines Medikaments zur Behandlung von Atherosklerose, wobei etwa 800 mg bis etwa 1 Gramm Nikotinsäure und Verbindung E einmal täglich für einen Zeitraum im Bereich von 3 - 7 Tagen bis etwa 2 Monaten an einen Patienten verabreicht werden und anschließend etwa 2 Gramm Nikotinsäure und Verbindung E einmal täglich für die Dauer der Therapie an den Patienten verabreicht werden, wobei Verbindung E in einer Menge verabreicht wird, die eine bedeutende Hautgefäßerweiterung wirksam minimiert oder eliminiert.

12. Die Verwendung nach Anspruch 11, ferner umfassend einen HMG-CoA-Reduktase-Inhibitor.

13. Die Verwendung nach Anspruch 11, wobei die Kombination in Form einer 2-Schicht-Tablette vorliegt, umfassend eine erste Schicht, die etwa 1 g Nikotinsäure in einer Matrix zur Freisetzung mit konstanter Geschwindigkeit enthält, und eine zweite Schicht, die etwa 20 mg Verbindung E enthält.

14. Die Verwendung nach Anspruch 11, wobei die Kombination in Form einer 2-Schicht-Tablette vorliegt, umfassend eine erste Schicht, die etwa 1 g Nikotinsäure in einer Matrix zur Freisetzung mit konstanter Geschwindigkeit enthält, und eine zweite Schicht, die etwa 20 mg Verbindung E und etwa 10 mg, 20 mg oder 40 mg Simvastatin enthält.

15. Die Verwendung nach Anspruch 11, wobei die Verwendung die einmal tägliche Verabreichung einer feststehenden Kombination, die etwa 800 mg bis etwa 1 Gramm Nikotinsäure, etwa 20 mg Verbindung E und gegebenenfalls etwa 20 mg Simvastatin umfasst, für einen Zeitraum, der von etwa 3 Tagen bis etwa 2 Monate reicht, gefolgt von der einmal täglichen Verabreichung von zwei feststehenden Kombinationsdosisformen, die insgesamt etwa 2 Gramm Nikotinsäure, etwa 40 mg Verbindung E und gegebenenfalls 40 mg Simvastatin umfassen, für die Dauer der Therapie umfasst.

## Revendications

1. Combinaison d'acide nicotinique et de Composé E de la formule suivante: 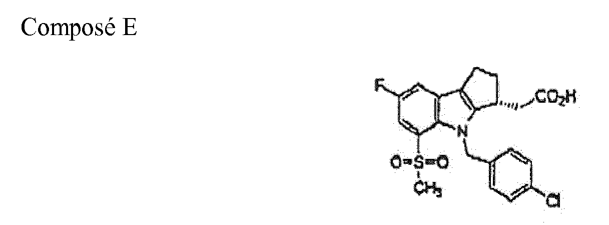
ou d'un sel ou solvate pharmaceutiquement acceptable de celui-ci, à utiliser pour traiter l'athérosclérose, pour élever les taux sériques de HDL ou pour traiter la dyslipidémie, en l'absence de bouffée de chaleur substantielle, où d'environ 800 mg à environ 1 gramme d'acide nicotinique sont administrés à un patient une fois par jour pendant une période allant de 3-7 jours à environ 2 mois et par la suite, d'environ 1,6 grammes à environ 2 grammes d'acide nicotinique sont administrés au patient une fois par jour pendant la durée du traitement.

2. Combinaison à utiliser selon la revendication 1 comprenant en outre de la simvastatine.

3. Combinaison à utiliser selon la revendication 1 ou 2, dans laquelle l'acide nicotinique allant d'environ 800 mg à environ 1 gramme, est sous forme d'un comprimé bicouche à libération prolongée, constitué d'une première couche contenant de l'acide nicotinique dans une matrice de libération prolongée et d'une deuxième couche contenant une quantité efficace de Composé E.

4. Combinaison à utiliser selon la revendication 1 ou 2, dans laquelle les 1,6 grammes à 2 grammes d'acide nicotinique sont sous forme de deux comprimés bicouche à libération prolongée, constitués chacun d'une première couche contenant environ de 800 mg à environ 1 gramme d'acide nicotinique et d'une deuxième couche contenant une quantité efficace de Composé E.

5. Combinaison à utiliser selon la revendication 3 ou 4, dans laquelle la deuxième couche contient en outre environ 20 mg de simvastatine.

6. Combinaison à utiliser selon la revendication 3 ou 4, dans laquelle la deuxième couche contient une quantité efficace d'atorvastatine.

7. Combinaison à utiliser selon la revendication 1, comprenant en outre un inhibiteur de la HMG-CoA réductase.

8. Combinaison à utiliser selon la revendication 1, où la combinaison est sous forme d'un comprimé bicouche constitué d'une première couche contenant environ 1 g d'acide nicotinique dans une matrice de libération prolongée et d'une deuxième couche contenant environ 20 mg de Composé E.

9. Combinaison à utiliser selon la revendication 1, où la combinaison est sous forme d'un comprimé bicouche constitué d'une première couche contenant environ 1 g d'acide nicotinique dans une matrice de libération prolongée et d'une deuxième couche contenant environ 20 mg de Composé E et environ 10 mg, 20 mg ou 40 mg de simvastatine.

10. Combinaison à utiliser selon la revendication 1, où l'utilisation comprend l'administration une fois par jour d'une combinaison fixe qui est constituée d'environ 800 mg à environ 1 gramme d'acide nicotinique, d'environ 20 mg de composé E et optionnellement d'environ 20 mg de simvastatine, pendant une période d'environ 3 jours à environ 2 mois, suivie par l'administration une fois par jour de deux formes de présentation de combinaison fixe comprenant au total environ 1,6 grammes à environ 2 grammes d'acide nicotinique, environ 40 mg du composé E et optionnellement 40 mg de simvastatine, pendant la durée du traitement.

11. Utilisation d'une combinaison d'acide nicotinique et de Composé E de la formule suivante: 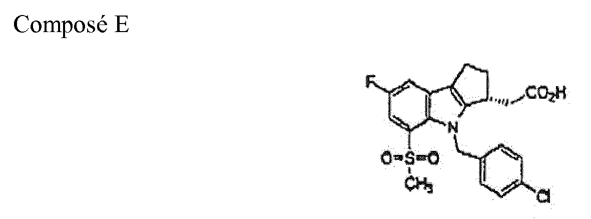 ou d'un sel ou solvate pharmaceutiquement acceptable de celui-ci, dans la fabrication d'un médicament pour traiter l'athérosclérose, où d'environ 800 mg à environ 1 gramme d'acide nicotinique et de composé E sont administrés à un patient une fois par jour pendant une période allant de 3-7 jours à environ 2 mois, et par la suite environ 2 grammes d'acide nicotinique et de composé E sont administrés au patient une fois par jour pendant la durée du traitement, où le composé E est administré en une quantité qui est efficace pour minimiser ou éliminer des bouffées de chaleur substantielles.

12. Utilisation selon la revendication 11, comprenant en outre un inhibiteur de la HMG-CoA réductase.

13. Utilisation selon la revendication 11, où la combinaison est sous forme d'un comprimé bicouche constitué d'une première couche contenant environ 1 g d'acide nicotinique dans une matrice de libération prolongée et d'une deuxième couche contenant environ 20 mg de Composé E.

14. Utilisation selon la revendication 11, où la combinaison est sous forme d'un comprimé bicouche constitué d'une première couche contenant environ 1 g d'acide nicotinique dans une matrice de libération prolongée et d'une deuxième couche contenant environ 20 mg de Composé E et environ 10 mg, 20 mg ou 40 mg de simvastatine.

15. Utilisation selon la revendication 11, où l'utilisation comprend l'administration une fois par jour d'une combinaison fixe qui est constituée d'environ 800 mg à environ 1 gramme d'acide nicotinique, d'environ 20 mg de composé E et optionnellement d'environ 20 mg de simvastatine, pendant une période d'environ 3 jours à environ 2 mois, suivie par l'administration une fois par jour de deux formes de présentation de combinaison fixe comprenant au total environ 2 grammes d'acide nicotinique, environ 40 mg de composé E et optionnellement 40 mg de simvastatine, pendant la durée du traitement.
